# EUROPEAN PATENT SPECIFICATION

(11) **EP 3 731 201 B1**
(45) Date of publication and mention of the grant of the patent: **09.11.2022**
(21) Application number: 19170924.5
(22) Date of filing: 24.04.2019
(51) Int. Cl.: G08B 21/04, G08B 25/10, G08B 21/06, G05B 19/042

(54) **A SUPPORT SYSTEM FOR AN OPERATOR**
UNTERSTÜTZUNGSSYSTEM FÜR EINEN BEDIENER
SYSTÈME DE SUPPORT POUR UN OPÉRATEUR

(43) Date of publication of application: 28.10.2020
(73) Proprietor: ABB Schweiz AG, 5400 Baden (CH)
(72) Inventor: Lymperopoulos, Ioannis, 5405 Baden-Dättwil (CH); Cortinovis, Andrea, 5405 Baden-Dättwil (CH); Mercangoez, Mehmet, 5405 Baden-Dättwil (CH)
(74) Representative: Kolster Oy Ab

(56) References cited:
- EP-A1- 3 012 701
- EP-A1- 3 459 807
- WO-A1-2018/231561
- WO-A2-2014/057308
- US-A1- 2011 276 165

## Description

### FIELD

The present invention relates to a support system for an operator monitoring and controlling one or more industrial processes.

### BACKGROUND ART

The evolvement of networking between computers and measurement devices, especially different sensors, capable of communicating without user involvement, has increased the amount of data collected on equipment and processes. By way of example, it is not unheard of to have thousands of sensors and elements of decision-making, monitoring and controlling aspects of a process and equipment within an industrial plant. The collected data, or at least some of it, is typically transmitted to a control system, which is usually a distributed control system, and displayed via graphical user interfaces (GUIs) in a control room for one or more human operators. The human operators can view and control, for example issue process commands, any part of the process via human-machine interfaces, such as screens and consoles, whilst retaining a plant overview to maintain safe and efficient operation of the plant. A delayed control action will decrease production capacity and may cause unscheduled downtime of the process and/or poor quality.

EP 3012701 discloses a method for managing operator alertness and enhancing operator effectiveness for industrial control systems. The method includes receiving activity data from a plurality of sensors associated with at least a portion of an industrial process system. The method also includes monitoring the activity data to identify a period of inactivity of all of the plurality of sensors. The method also includes issuing an alarm responsive to identifying the period of inactivity.

EP 3459807 discloses an operator monitoring system for use in a ground based vehicle. The operator system includes a monitoring system to collect information regarding one or more characteristics of the operator during operation of the vehicle, a core platform configured to determine whether the one or more characteristics corresponds to a fatigue indicator, a response system configured to generate a warning based at least in part on the fatigue indicator and an interface to present the warning to the operator.

US 2011/0276165 discloses an integrated expert system for identifying abnormal events in an industrial plant. The expert system integrates a model-based expert system with a rule-based expert system. The model-based expert system receives data on the operating conditions of a process unit in the industrial plant and calculates one or more results that determine whether the process unit is operating abnormally. The rule-based expert system also receives data on the operating conditions of the process unit and applies its rules to the process data. The rule-based expert system also applies its rules to the one or more results generated by the model-based expert system.

WO 2014/057308 discloses an apparatus for maintaining alertness of a driver of a motor vehicle. The apparatus periodically generates an audible alert signal to which the driver responds by pressing a button on the vehicle's steering wheel. The response time of the driver to the signal is monitored and if an increase is detected, the repetition rate of the alert signal is increased. The repetition rate may be further modified by taking into account vehicle driving conditions which may indicate a risk of boredom in the driver.

WO 2018/231561 discloses a method for identifying, visualizing, and triggering workflows from auto-suggested actions to reclaim lost benefits of model-based industrial process controllers. The method includes obtaining data associated with operation of an industrial process controller and identifying impacts of operational problems of the industrial process controller. The method also includes generating a graphical display for a user, where the graphical display presents one or more recommended actions to reduce or eliminate at least one of the impacts of at least one of the operational problems. The method further includes triggering at least one of the one or more recommended actions based on input from the user.

### SUMMARY

An object of the present invention is to provide a mechanism suitable for providing support to human operators. The object of the invention is achieved by a method, a computer program product, equipment and a system, which are characterized by what is stated in the independent claims. Further embodiments of the invention are disclosed in the dependent claims.

### BRIEF DESCRIPTION OF THE DRAWINGS

In the following, exemplary embodiments will be described in greater detail with reference to accompanying drawings, in which
Figure 1 shows a simplified architecture of a system;
Figure 2 illustrate an example information flow;
Figures 3 to 11 are flow charts illustrating examples of functionalities; and
Figure 12 is a schematic block diagram.

### DETAILED DESCRIPTION OF SOME EMBODIMENTS

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments.

The present invention is applicable to any industrial control room, in which operation settings may be adjusted by a human operator, or automatically set adjustments manually overridden by the human operator. The operator settings may be, for example, for a processing system and/or for an industrial manufacturing related process and/or for a system for a technical process. A non-limiting list of examples includes control rooms for power plants, manufacturing plants, chemical processing plants, power transmission systems, mining and mineral processing plants, upstream oil and gas systems, data centers, ships, and transportation fleet systems.

Different embodiments and examples are described below using single units, models, equipment and memory, without restricting the embodiments/examples to such a solution. Concepts called cloud computing and/or virtualization may be used. The virtualization may allow a single physical computing device to host one or more instances of virtual machines that appear and operate as independent computing devices, so that a single physical computing device can create, maintain, delete, or otherwise manage virtual machines in a dynamic manner. It is also possible that device operations will be distributed among a plurality of servers, nodes, devices or hosts. In cloud computing network devices, computing devices and/or storage devices provide shared resources. Some other technology advancements, such as Software-Defined Networking (SDN), may cause one or more of the functionalities described below to be migrated to any corresponding abstraction or apparatus or device. Therefore, all words and expressions should be interpreted broadly, and they are intended to illustrate, not to restrict, the embodiment.

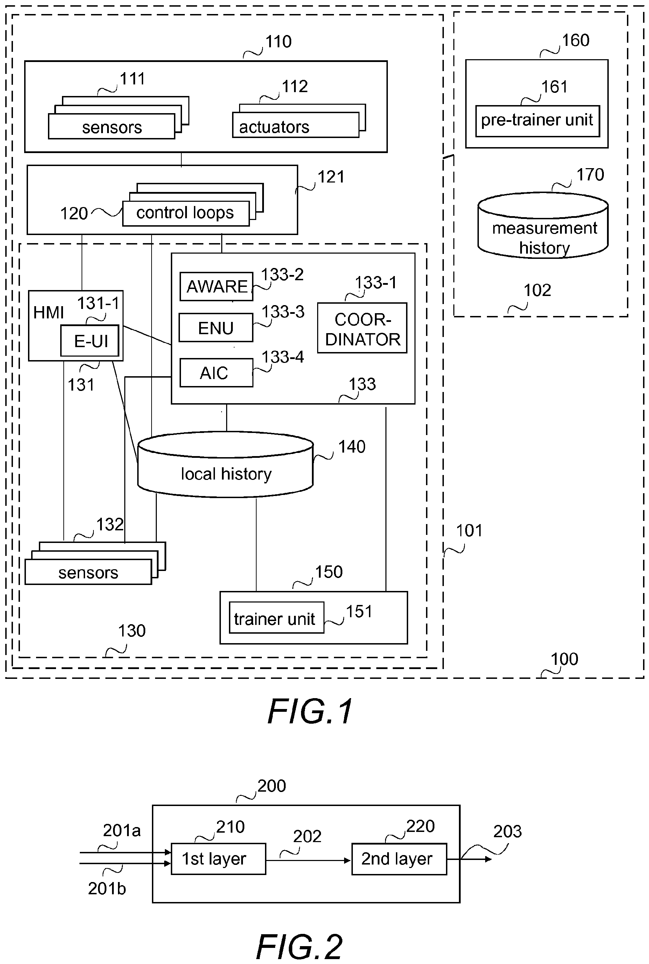

A general exemplary architecture of a system is illustrated in Figure 1. Figure 1 is a simplified system architecture only showing some equipment (apparatuses, devices, nodes) and functional entities, all being logical units whose implementation and/or number may differ from what is shown in Figure 1. The connections shown in Figure 1 are logical connections; the actual physical connections may be different. It is apparent to a person skilled in the art that the systems also comprise other equipment, functional entities and structures.

In the illustrated example of Figure 1, a system 100 comprises an industrial process system 101 and an offline site 102.

The industrial process system 101 depicts herein any process, or process system, including different devices, machines, apparatuses, equipment, and sub-systems in an industrial plant, examples of which are listed above. Further examples include pulp and paper plants, cement plants, metal manufacturing plants, refineries and hospitals. However, the industrial process system is not limited to the examples listed but covers any process that involves technical considerations and is not purely a business process. The industrial process system 101 comprises one or more processes 110 (only one is illustrated), controlled, for example, by control loops 120 forming a part 121 of a control system, or one or more parts of one or more control systems. It should be appreciated that term control covers herein also supply chain management, service and maintenance. The control loops 120 measure values for process variables of the processes 110 through sensors 111 and manipulate the process 110 through actuators 112, either automatically or according to operator inputs received from a human operator via one or more human-machine interfaces (HMI) 131 in a control room 130. The control loops 120 may be open control loops or closed control loops, and the control system 121 may be a distributed control system or a centralized control system.

The control room 130 refers to a working space/environment of one or more human operators. The control room 130 is for monitoring and/or controlling, including manipulating and adjusting, the one or more industrial processes 110, on the site and/or remotely. In other words, the control room 130 depicts a monitoring and/or controlling system (sub-system) that may be implemented by different devices comprising applications that analyse the data, or some pieces of the data, for controlling purposes in real-time, for example. A non-limiting list of examples of control rooms include power plant control rooms, process control rooms, grid control centers, mine central control rooms, oil and gas command centers, rail operating centers, traffic management centers, marine fleet handling centers, data center control rooms and hospital capacity command centers.

A wide range of applications exists for automation control and monitoring systems, particularly in industrial settings. The analysis may include outputting alarms, disturbances, exceptions, and/or outputting, after determining, different properties relating to the measurement data, such as a minimum value and a maximum value, and/or outputting, after calculating, different key performance indicators. The control room 130 comprises one or more human-machine interfaces (HMI) 131 to output the different outputs to one or more human operators in the control room, so that a human operator may detect anomalies (non-anticipated behavior, deterioration of performance, etc.), manipulate, control or adjust the one or more processes, for example by inputting user inputs via one or more user interfaces. Further, the human-machine interfaces 131 are configured to measure online, i.e. track, human operator's interactions with the one or more process and store the interactions (user inputs), for example to a local database 140. The measured interactions may indicate how long it took from the user to actually input something once user input was prompted (requested) and coherency of user inputs. The interactions may be stored as anonymized data, preferably in such a way that they constitute human operator -specific private information, accessible by the human operator in question as his/her data and accessible to others as anonymous information or metadata.

In the illustrated example of Figure 1, the control room 130 comprises, for a support system for an operator (a human operator), one or more different sensors 132, one or more engagement user interfaces 131-1 (E-Ul), and equipment 133 which is called herein an artificial intelligence operator, AI operator. Further, in the illustrated example, the control room also comprises the local database 140 (local history) and local offline equipment 150. However, the local database 140 and/or the local offline equipment 150 may locate in a location remote to the control room, and the local database 140 may locate in a different location than the local offline equipment 150.

An engagement user interface 131-1 is a separate interface, or at least distinct environment from the monitoring environment, that the one or more human-machine interfaces 131 (HMI) comprises for engagement purposes. Since it is a separate interface from interface(s) used for monitoring and controlling, a human operator can easily distinguish the engagement view (training view or simulation view, or emulation view) from the real view of the process the human operator is monitoring. Naturally, also user inputs (interactions) to the engagement user interface are tracked (measured) and stored, for example to the local database 140, preferably as anonymized data.

The one or more different sensors 132 collect data on the human operator, or human operators, in the control room 130. A sensor may be installed in the control room, or it may be a wearable sensor. A non-limiting list of sensors 132 include a visual sensor, like a camera, an audio sensor, like an audio recorder, and biometric sensors for measuring different physiological values, like blood pressure, body temperature and heart beat rate. Further, the one or more different sensors 132 in the control room may also comprise sensors that collect environmental information on the control room, such as room temperature, humidity, lightness, or other information on the microclimate in the operator room. The sensor data collected on the human operator is inputted to the AI operator 130 and also stored to the local database 140, preferably as anonymized data.

In the illustrated example of Figure 1, the AI operator 133 comprises, as sub-systems of the support system, a coordinator unit 133-1, an awareness unit 133-2 (AWARE), an engagement unit 133-3 (ENU) and an artificial intelligence companion unit 133-4 (AIC). The coordinator unit 133-1 is configured to coordinate operations of the other units. The awareness unit 133-2 is configured/trained to assess the human operator's alertness level. The engagement unit 133-3 is configured/trained to output interactive engagement sessions via the engagement user interface 131-1. The artificial intelligence companion unit 133-4 may be configured/trained to detect anomalies and/or create/generate real-time or near real-time predictions at least from values of the process variables measured by means of the sensors 111, and/or provide different suggestions to the human operators. The AI operator and its units will be described in more detail below with Figures 2 to 10.

The local database 140 is configured to store local history, i.e. information collected on the one or more processes 110, user inputs to the human-machine interface 131, including the engagement user interface 131-1, and sensor measurement results of the one or more sensors 132 in the control room. In other words, the local history comprises data specific to the control room, the operators working in the control room and the controlled process/plant.

The local offline equipment 150 comprises a trainer unit 151 configured to at least train a model, or finalize training of a pre-trained model, for the awareness unit 133-2, and retrain the model, using local history in the local data storage 140. The local offline equipment 150 may comprise trainer units for other trained models used in the other units of the AI operator 133. Naturally, the equipment 150 may be an online equipment, or integrated with the offline site 102.

The offline site 102 of Figure 1 further comprises an offline equipment 160 with a pre-trainer unit 161 configured to create at least a pre-trained model for the awareness unit 133-2 using measurement history in a data storage 170 in the offline site 102. The measurement history in the data storage 170 may comprise data collected, and possibly stored to corresponding local data storages, at different sites/plants and control rooms. In other words, the anomalies, measured values of the process variables and control room variables, including values measured on the human operator, may be stored to the local data storage 140 and to the data storage 170 (archive for measurement results).

Further, the offline equipment 160 may comprise trainer units and/or pre-trainer units for other trained models that may be used in the other units of the AI operator 133. Naturally, the equipment 160 may be an online equipment, at least in the sense that measurements results may also be inputted to the equipment 160 in real time, or almost real time.

To finalize a pre-trained model using the data specific to the control room and the controlled process/plant customizes the awareness unit for the specific environment, whilst the pre-training using bigger amount of data stored to the archive data storage 170 provide better accuracy and allows faster convergence.

The AI operator 133 and/or the local offline equipment 150 and/or the offline equipment 160 may comprise customized computational hardware for machine learning applications and/or for artificial neural networks, for extra efficiency and real-time performance.

The local data storage 140, depicting one or more data storages, and the archive data storage 170, depicting one or more data storages, may be any kind of conventional or future data repository, including distributed and centralized storing of data, managed by any suitable management system. An example of distributed storing includes a cloud-based storage in a cloud environment (which may be a public cloud, a community cloud, a private cloud, or a hybrid cloud, for example). Cloud storage services may be accessed through a co-located cloud computer service, a web service application programming interface (API) or by applications that utilize API, such as cloud desktop storage, a cloud storage gateway or Web-based content management systems. In other words, a data storage 140, 170 may be a computing equipment, equipped with one or more memories, or a sub-system, or an online archiving system, the sub-system or the system comprising computing devices that are configured to appear as one logical online archive (historian) for equipment (devices) that store data thereto and/or retrieve data therefrom. However, the implementation of the data storage 140, 170 the manner how data is stored, retrieved and updated, i.e. details how the data storage is interfaced, and the location where the data is stored are irrelevant to the invention. It is obvious for one skilled in the art that any known or future solution may be used.

Figure 2 illustrates an example of a logical structure of a trained model for the awareness unit, and a basic principle how the awareness unit produces one or more alertness levels. The trained model illustrated utilizes, i.e. is based on, machine learning, and more precisely on deep learning. If inputs are received from different sources, the trained model may be based on a multimodal deep learning method. Naturally, any other machine learning, such as image learning, rule-based learning and explanation-based learning, may be used.

The awareness unit 200 illustrated in Figure 2 is based on a deep neural network. The illustrated structure is highly simplified illustrating only two layers, although a deep neural network comprises several layers: one or more input layers, one or processing layers, which may include one or more recurrent layers, one or more neural layers, etc., one or more output layers for determining quantities of interest and one or more decision layers for taking decisions according to created outputs from the output layer.

Referring to Figure 2, the illustrated awareness unit comprises a first layer 210, depicting input layer(s) and processing layer(s), and a second layer 220, depicting output layer(s) and decision layer(s). Two or more inputs 201a, 201b depicts herein inputs 201a representing online measurement results relating to the control room/human operator and inputs 201b representing online measurement results from the process. The inputs 201a representing online measurement results relating to the control room and/or human operator comprise at least interaction data. The interaction data refers to data collected when the human operator has interacted with the process/plant. The interaction data may comprise switching between screens, set point changes, manual on/off switching, etc., and it may be obtained from information collected on the one or more processes . Naturally, the number of interactions within a certain time period is interaction data, and vary from process to another. The inputs 201b representing online measurement results from the process comprise at least process response data. The process response data refers to indirect results (values) of the human operator's control actions, collected (measured) on the process. In other words, values in the process response data are values which the human operator cannot directly set (or otherwise manipulate/control), only to observe. Following example is just to more clarify what is meant by process responses, i.e. process response data. A motor is running, speed and temperature are measured and displayed as online measurement results to a human operator. The human operator can directly set a speed of a motor to a certain value, and the speed values measured after that will be the speed set by the human operator but temperature values measured after that are process responses, the human operator cannot set the temperature. If the human operator wants to increase the temperature by a certain amount, the human operator needs to know how to change the speed, and hope that the speed change causes the wanted temperature increase.

Naturally, also other online measurement results from the one or more processes may be inputted to the awareness unit.

The first layer 210 processes the two or more inputs 201a, 202b into interpretable features 202. The interpretable features 202 are then input to the second layer 220. The second layer processes 220 the interpretable features to one or more alertness levels 203, for example to one or more indices, outputted from the awareness unit. The one or more alertness levels may be outputted to the coordinator unit, or to the coordinator unit and via one or more human-machine interfaces also to the one or more human operators. Further, the alertness levels may be outputted, preferably anonymized, to a supervisor who is responsible for safety and well-being of the human operators, and scheduling of work. The supervisor may use the alertness levels to request emergency support, release extra capacity (high alertness) to be used somewhere else, schedule working shifts according to human operators' optimal time-shifts, etc., all being measures that in the long run increase the productivity and shortens shut down times of the process.

The interaction data may be used as such as interpretable features 202. The process data may also be used as such as interpretable features 202 and/or the interpretable features 202 from the process data may comprise deviations from expected value, for example.

The inputs 201a representing online measurement results relating to the control room/human operator may also comprise, as further inputs, sensor data measured by the one or more sensors described above with Figure 1. A non-limiting list of one or more further inputs 201ato the awareness unit, include visual data, for example facial expressions and body movements, audio data, different biometrics measured on the body of the human operator, such as hear beat rate, body temperature, and environmental conditions (microclimate of the operator room), like room temperature, and humidity. The data measured/captured on the human operator assists in augmenting the accuracy of the interaction data and process response data. Further, the environmental conditions have a substantial impact on the alertness of the human operator, so inputting also the environmental conditions further increases the accuracy.

A non-limiting list of interpretable features 202 from the visual data include emotional state, body pose and eye gaze tracking. A non-limiting list of interpretable features 202 from the audio data include conversation analysis and sound distractions. A non-limiting list of interpretable features 202 from the biometrics includes stress level and body comfort. The environmental conditions may be used as such as interpretable features 202.

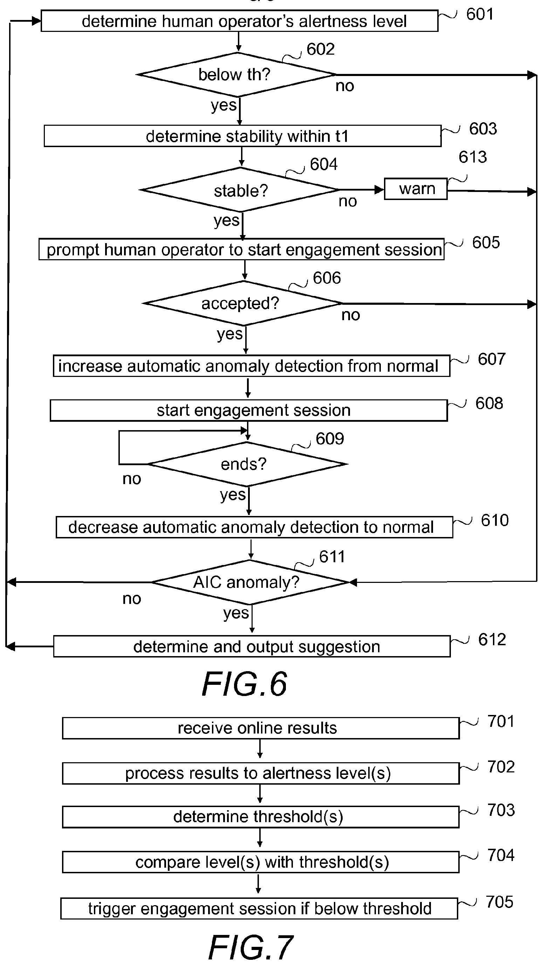

A non-limiting list of alertness levels include an index value for engagement, an index value for stress, an index value for confidence and an index value for alarmed. For example, each of the indices may be any numerical value between 0 to 100. It should be appreciated that instead of index values alertness levels the output may indicate the alertness level in another way. For example, the alertness level may be simply be indicated to be either "not focused" or "focused and alert", indicated by one bit (for example 0 meaning "not focused"). The alertness level may be "not focused", if the frequency of the interaction is below a threshold determined by the awareness unit, or preset to the awareness unit. Further examples are described below with Figure 7.

It should be appreciated that the awareness level is assessed on individual basis. If there are two or more human operators in the same control room, there are several ways how they are differentiated. For example, face-recognition software can distinguish them, if sensors for visual data are installed and used in the control room, or each operator may have his/her own tag for identifying purposes, the location where they are may be used to differentiate human operators, etc.

In a summary, the awareness unit preferably fuses data streams from different sensors and generates real-valued outputs that assess the situational awareness of the human operator (engagement, confidence, stress, level of alarm) in real time. However, as a minimum requirement, the human operator's interactions and process response data are needed to generate an indication of the alertness level in real time.

Compared to prior art solutions, like in pilot and driver assistant solutions, in which the performance monitoring is rather straightforward, determining the alertness level for a process control requires more sophisticated design; in the pilot and driver assistant solutions a low altitude or lane crossing are easy to detect whereas in industrial process there is no such easily measurable and detectable limit which indicates a low alertness level of the human operator.

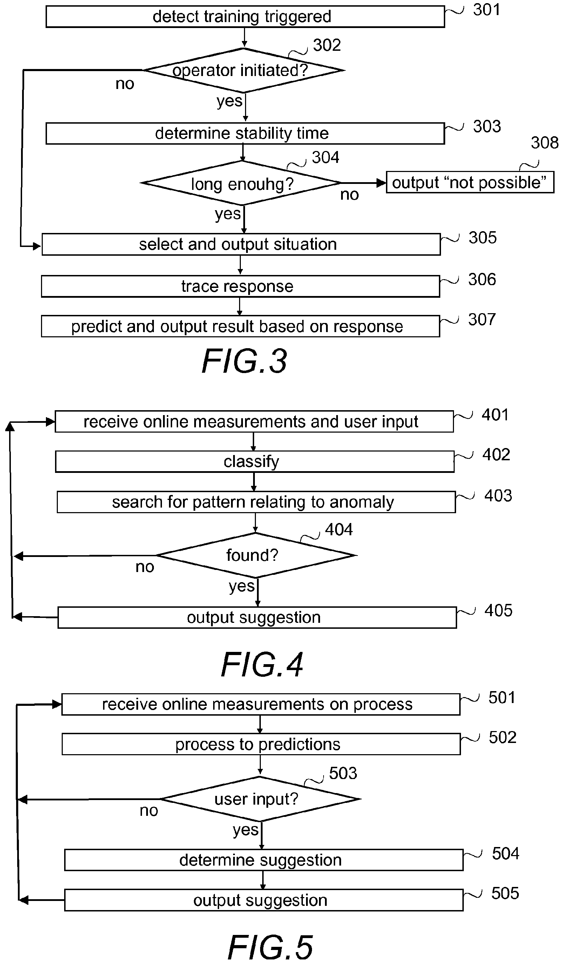

Figure 3 illustrates an example functionality of the engagement unit during an engagement session comprising one engagement event. A non-limiting list of examples of an engagement session include a computer-based automated interactive training session relating to the process, a computer instructed physical activity session, a computer narrated audio reporting session about the performance of the operation of the process, a computer narrated visual reporting session about the performance of the operation of the process, an application-based interactive training session with gamification, the training session relating to the process, and any combination thereof.

The engagement unit, when configured to provide training sessions relating to the process, may be based on a data driven model of the process 102, which has classified past anomalies, including unexpected incidents, with corresponding human operator control actions, and results caused by the control actions. The data driven model may be created by applying machine learning to the data in the local data storage, or in the archive data storage for similar processes, for example. The data driven model may be based on multi-level analysis to provide a detailed insight, at several levels and/or scales, into the process behavior. In other words, the data driven model may provide a mapping/function from input, which comprises one or more of human operator's control actions, environment information, current status of the process/plant and configuration of the process/plant, to process/plant output or next state or key performance indicators. Further, such engagement unit may be implemented by applying gamification. In the gamification, game-design elements and game principles are applied in non-game contexts, the game-design elements comprising scoring, for example.

In the example illustrated in Figure 3, an interactive training session with one training event relating to the process is used as an example of engagement sessions without limiting the example and its implementations to the training sessions. Further, it is assumed that the engagement unit is configured to provide training events, by selecting amongst past situations (anomalies) one and prompting the user to provide a solution, and then outputting an optimal solution. A virtual environment provided by the engagement unit in training sessions relating to the process may be seen as a digital twin of the process (or plant). The digital twin in turn provides an emulation of process dynamics for increased realism.

Referring to Figure 3, the process starts, when it is detected in step 301 that a training is triggered. The detection may be based on receiving a user input indicating "I want to train", or an internal input within the AI operator, the internal input being caused by the awareness unit detecting a low alertness level or the artificial intelligent companion detecting that there will be/has been a long inactivity time, for example.

If the training is operator initiated (step 302: yes), i.e. a corresponding user input has been received, an estimated stability time of the process is determined in step 303. The stability time is an estimation how long the training can take without withholding the human operator's attention from his/her actual task. More detailed explanation how the stability time may be determined is below with Figure 6. In the illustrated example, the stability time check is not performed if the training is triggered by the internal input, since it is assumed, that the training is triggered by the internal input only if a stability time is long enough. However, in another implementation the stability time will be checked also when the training is triggered by the internal input.

If the stability time is long enough (step 304: yes) or if the training is triggered by the internal input (step 302: no), a training event, i.e. a situation requiring human operator involvement, is selected in step 305 amongst a set of training events in the local data storage, or in the engagement unit, for the training session and the situation is outputted in step 305 to the human operator. The training events in the set of training events may comprise, for example, events obtained automatically, using principles described below with Figure 4, and/or from training kits designed for training purposes and/or defined by the human operators. The training event may be selected randomly, in turns, depending on the frequency such anomaly takes place in the real process, the importance of the training event to the operation of the process/plant, the time it takes to complete the training event, the time the training event was last selected, and/or on the basis how difficult the training session is, when judged by the successfulness of responses, for example. Further, the human operator may be prompted to select the training event amongst all training events, or amongst a sub-set of training events, the sub-set being selected using, for example, the same principles described above. As described with Figure 1, the situation is outputted in a specific training user interface, which provides a distinct environment from the active operator view on the process(es) the operator is monitoring so that the emulated (simulated) situation is not mixed with current situation of the real process (es). While outputting (replaying) the situation, the engagement unit prompts the human operator to perform one or more actions (control actions) that can resolve the selected situation, both in terms of improving performance and warranting safety. The one or more actions performed by the human operator, i.e. the response received via the engagement user interface, is traced in step 306. The tracing includes storing the responses, preferably as encrypted and anonymized to provide data protection for the human operators.

The response is then processed in step 307 to a prediction on how the process would turn in response to the action if the situation would be a real situation, and the result is outputted in step 307 to the human operator. Alternatively, or in addition to, an optimal solution may be outputted, or the human operator may be provided with other kind of tips (alternative solutions) how to react to the situation. If gamification is implemented, a score may be provided, and ranked in comparison with other human operators being trained, preferably even with the same situation.

Naturally, after the result is outputted, the human operator may be prompted to select, whether to continue the training session, and if the input is to continue, the process returns to step 303 to determine the stability time.

If the stability time is not long enough (step 304: no), in the illustrated example the human operator is informed by outputting in step 308 information indicating that the training is not possible now.

By the training, using situations that have happened in the process that is being monitored, the mental state of the human operator is kept in proximity with his/her control tasks. Further, it provides a convenient way to transfer knowledge to the human operator on control actions of other human operators, and the best ways to solve the situations, thereby increasing experience of the human operator. Further, the training provides also training for rare situations, i.e. anomalies that happen in real life seldom but for which it is good to be prepared. In other words, the training facilitates teaching complex tasks with no or minimal expert knowledge of a human operator teaching another human operator. This is especially useful in larger and more complex industrial plants, with more complex control applications, when there is more data to interpret and/or more variety of alarms, since it decreases a probability of a non-optimal control action. A non-optimal control action in turn will decrease production capacity and may cause unscheduled downtime of the process and/or poor quality. If the gamification is applied, the human operator may be more motivated to train, especially when everything in the process is going well without anomalies and without active interaction with the process.

Figures 4 and 5 illustrate examples of different functionalities of the artificial intelligence companion unit. Depending on an implementation, the artificial intelligence companion unit may be configured to implement both functionalities, or one of them.

Referring to Figure 4, the artificial intelligent companion unit may be trained to be a trained classifier, which when it receives online measurements on the process and the human operator's interactions (tracked user inputs) in step 401, it classifies in step 402 inputs relating to human operator control actions. Such inputs include those values measured by sensors, which triggered a human operator control action, the human operator control action, time it took to take the control action, what is/was the result of the human operator control action, and preferably also measured values on the human operator and/or the control room. In other words, at least anomalies with corresponding user input (control action) are classified. A non-limiting list of control actions, i.e. user inputs, include change of various set point values, switching equipment on or off, switching between different production modes, and triggering or dispatching maintenance activities. Then, for on-time detection, a similar pattern, i.e. a pattern relating to the anomaly, is searched in step 403 for amongst the stored classified results. A pattern may be an event captured in a time series of signals, or an event, which requires user action and which exists in current and preceding measurements. If any similar pattern is found (step 404: yes), a suggestion, based on the previous actions and results, is outputted in step 405 via one or more human-machine-interface , to support the human operator in decision making what control action(s) to perform. Then the process continues to step 401 to receive information. If no similar pattern is found (step 404: no), the process continues to step 401 to receive information.

It should be appreciated that the engagement unit may be configured to perform corresponding classification, and/or use the data classified by the artificial intelligent companion unit for training events, or other type of engagement events.

The data classified as described can be used by the artificial intelligence companion unit and the engagement unit to continuously update the corresponding units by imitation learning, for example.

The artificial companion unit may further be configured to provide predictions on the monitored industrial process. For example, the artificial intelligence companion unit may comprise online prediction equipment described in a European patent application number 18212177 for anomaly detection and/or for predictions, possibly further configured to output suggestions to the human operator, and/or to classify inputs. The European patent application number 18212177 is assigned to the same applicant, has been filed on 13 December 2018 and is hereby incorporated by reference. Naturally, any other data driven model trained to generate on-time, optimized suggestion based on previous human operator control actions with respective process responses in similar situations may be used.

Referring to Figure 5, when online measurement results of the industrial process are received (step 501), the artificial intelligence companion unit processes them in step 502 to one or more predictions on the process. Then it is determined, whether the prediction indicates an anomaly that will require human operator input, i.e. user input. The human operator input may be required for each anomaly, or for certain type of anomalies.

If user input is required (step 503: yes), one or more suggestions on control actions to take are determined in step 504 and outputted in step 505. The suggestions outputted assist the human operator in decision making on whether and how to adjust the process. The suggestion may be a specific control action, instruction to move a valve position or change a motor speed, or shut down the system or start up a parallel process, for example. Since a prediction may alert the human operator about a critical situation with a suggestion how to overcome the critical situation before it happens, the human operator may perform control actions in advance so that the critical situation will not occur, which in turn increases productivity (efficiency of production) and shortens shut-down times, compared to solutions in which alerts are generated in response to the critical situation happening.

After outputting the one or more suggestion, or if it is determined that no user input is required (step 503: no), the process continues in step 501 by receiving online measurements.

Naturally, even though not explicitly mentioned, the predictions may be outputted, with the measured values, to the one or more human operators to indicate the current and future state, even when no anomalies are detected.

Figure 6 illustrates a functionality of the AI operator according to an example implementation illustrated in Figure 1. The functionality describes a continuous process, in which different steps may be parallel even though described for the sake of clarity as successive steps. Further, even though the steps are associated with the unit (sub-system) performing the step, the steps may be performed by other units, or the AI-operator may be one unit performing all the steps. Further, in the illustrated example normal control actions and human operator-process interactions are not shown.

Referring to Figure 6, the human operator's alertness level is determined, by the awareness unit, in step 601. Then it is checked in step 602, by the coordinator unit, whether or not the alertness level is below a threshold, i.e. whether or not the human operator is focused enough. The checking may include determining the threshold, as described below with Figure 7.

If the alertness level is below the threshold (step 602: yes), i.e. the human operator is not focused enough, the coordinator unit causes the artificial intelligence companion unit to determine in step 602, within time t1, the predicted stability of the process monitored by the human operator. The time t1 may be an estimate on the time one engagement event is assumed to take on average. The stability indicates a state in no anomality, or at least anomality requiring the human operator involvement, should take place. As long as the process/plant remains stable, the engagement event can be safely performed without taking the human operator's attention from his/her actual task. However, if the process/plant requires human operator input, or is in unstable state, or is expected to require human operator input or to enter unstable state soon (within the time t1, possibly added with some additional time), an engagement session should not be triggered.

If the prediction indicates that the process will be stable within the time t1 (step 604), the coordinator unit prompts in step 605 the human operator to start an engagement session. If an acceptance to start the engagement session is received, via a user interface, (step 606: yes), the coordinator unit increases in step 607 an automatic anomaly/fault detection from a normal to a more sensitive level. In other words, in an automatic anomaly /fault subsystem an increase is triggered. By doing so, it is ensured that the human operator will receive an alert, for example, so that he/she can more rapidly disengage from the engagement session if required because something unforeseeable happened. Further, the engagement session is started in step 608 by the coordinator unit in the engagement unit. The engagement session may be one of the engagement sessions described above with Figure 4, and comprise one or more engagement events. Once the engagement session ends (step 609: yes), the coordinator unit decreases in step 610 the automatic anomaly/fault detection back to the normal level. The levels may be based on probabilities of predictions of anomalies. For example, in a normal level any event requiring user action that is predicted to happen in the next 30 minutes with 30 % or more probability triggers a warning to the human operator but in a sensitive level the probability that triggers the warning may be 20 % or more.

Further, it is checked in step 611, by the coordinator unit, whether the artificial intelligence companion unit detected any anomaly (AIC anomaly). If an AIC anomaly is detected (step 611: yes), the artificial intelligence companion unit determines in step 612 a suggestion for control actions for the anomaly, and outputs in step 612 the suggestion, and preferably the anomaly as well unless not outputted when detected. Then the process illustrated continues to step 601 to determine the alertness level. Naturally, step 611 is performed as a background step all the time the process is run, and if an AIC anomaly is detected, the process proceeds immediately to step 612. For example, the engagement session (engagement event) may be paused or stopped automatically.

If no AIC anomaly is detected (step 611: no), the process illustrated continues to step 601 to determine the alertness level.

If the human operator is focused enough (step 602: no), no engagement session is suggested but the process proceeds to step 611 to check, whether there is an AIC anomaly.

If the process will not be stable within the time t1 (step 604: no), in the illustrated example the human operator's alertness is increased by warning in step 613 the human operator. The warning may be changing the background color of a display to another color and then back to the original color, providing a sound alert, etc. Then, or while still warning, the process proceeds to step 611 to check, whether there is an AIC anomaly. Also a supervisor and/or reserve personnel may be notified that the human operator has been warned, or that the human operator's alertness level is too low.

If the human operator declines the engagement session (step 606:no), the process proceeds to step 611 to check, whether there is an AIC anomaly.

An AI operator implementing the functionality described with above Figures provides an advanced support system for a human operator. The advanced support system assists the human operator with control tasks, increasing performance and long-term safety of equipment and personnel on the site where the industrial process is running. By means of the AI operator it is possible to minimize the number of sub-optimal decisions on what control actions to perform.

However, even a little less advanced support system, comprising the awareness unit and the engagement unit, provides advantages by keeping the human operator alertness level high enough by the engagement sessions. Further, when the engagement sessions are interactive training sessions relating to the process they increase the experience. Figure 7 illustrates an example functionality of a support system comprising the awareness unit and the engagement unit but no artificial intelligence companion unit.

Referring to Figure 7, when online measurements results, comprising at least the human operator's control actions and process responses to control actions, are received in step 701, they are processed in step 702 to one or more alertness levels (values) by the awareness unit, or corresponding machine learning based unit. Further, one or more thresholds defining minimum /maximum values indicating when the alertness is too low are determined in step 703. One or more of the thresholds may be a variable whose value depends on time of the day, for example and/or plant status (predicted status, or past status, or both). If there is enough operator-specific data available, the threshold may depend on the human operator data as well. The levels are then compared in step 704 with corresponding thresholds. If two or more levels are determined in step 702, each may be compared with a corresponding level, and it depends on the implementation how many of them needs to be below the threshold in order to determine that the alertness is too low. For example, if the frequency of the human operator's interactions is below a frequency determined by the trained model, the alertness is too low. In another example, if the delay of the human operator's response is more than an acceptable delay determined by the trained model for similar enough situations, for example, the alertness is too low. In a further example, if the process response to a control action deviates from an expected value determined by the trained model, the alertness is too low. In a still further example, if the operator's control action, or any interaction, is an outlier compared to how other operators have behaved in a similar situation, determined by the trained model, the alertness is too low. Alternatively, or in addition, from the two or more levels one indicator value may be calculated and compared with a threshold.

If the comparison in step 704 results that the alertness level is below the threshold, alertness of the human operator will be increased by triggering in step 705 an engagement session.

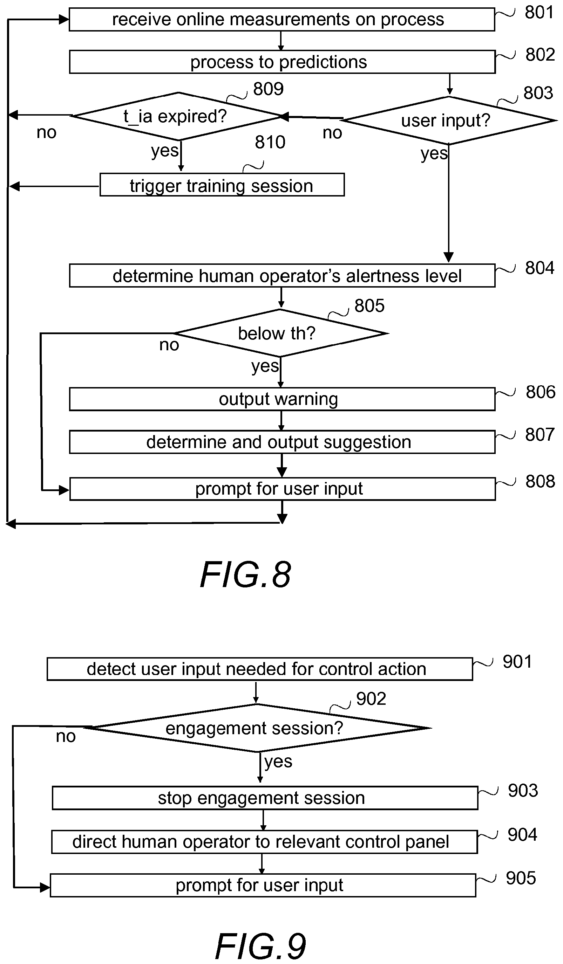

Another example of the little less advanced support system, comprising the awareness unit and the artificial intelligence companion unit, provides advantages by increasing the human operator alertness level when needed. Figure 8 illustrates an example functionality of such a support system. In the illustrated example, the support system is further configured to use so called fallow periods (stable periods in which it is assumed that no user interaction is needed) to increase human operator's experience by the training. Naturally, any other engagement session may be triggered instead of a training session.

Referring to Figure 8, when online measurement results of the industrial process are received (step 801), the artificial intelligence companion unit processes them in step 802 to one or more predictions on the process. Then it is determined in step 803, whether the prediction indicates an anomaly that will require human operator input, i.e. user input. The human operator input may be required for each anomaly, or for certain type of anomalies.

If user input is required (step 803: yes), the human operator's alertness level is determined, by the awareness unit, in step 804, or more precisely, its current value is obtained. Then it is checked in step 805, by the coordinator unit or by the artificial intelligence unit, whether or not the alertness level is below a threshold, i.e. whether or not the human operator is focused enough. The step may include determining the threshold, as described above with Figure 7.

If the alertness level is below the threshold (step 805: yes), i.e. the human operator is not focused enough, his/her alertness is increased in step 806 by outputting in step a warning 806. The warning may be similar to the warning described above with Figure 6. Further, it may include a more explicit warning, such as "your input for X may be required within five minutes". Further, one or more suggestions on control actions to take are determined in step 807 and outputted in step 807 to guide and assist the human operator to act properly when the user input (control action) is needed, as described above with Figure 5. Thanks to the warning and suggestions, the human operator will be more focused and know what to do and will be in a position to act in a timely manner in a proper way. This in turn increases productivity (efficiency of production) and shortens shut-down times, compared to solutions in which alerts are generated in response to the critical situation happening.

After outputting the one or more suggestions , or if the user is focused enough (step 805), the anomaly (event) happens in the example, and the user is prompt for user input in step 808, and the process continues in step 801 by receiving online measurements. Thanks to the warning and suggestions, the "non-focused" human operator will be more focused and know what to do and will be in a position to act in a timely manner in a proper way. This in turn increases productivity (efficiency of production) and shortens shut-down times, compared to solutions in which no prior warnings are created. It should be noted that in this example it is assumed that a focused human operator can act in a timely manner in a proper way. However, it is possible to provide suggestions also when the human operator is focused. (In other words, the process may continue from step 805 either to step 806 or to step 807.)

If no user input is required (step 803: no), in the illustrated example it is checked in step 809, whether an inactivity timer t_ia has expired. In the illustrated example the inactivity timer is maintained human operator -specifically as a background process and reset each time a human interaction is detected, the interactions meaning any interaction with the human-machine interface. The inactivity timer will expire when the inactivity time exceeds a limit, which may be a constant value or a variable and may be human operator -specific, and/or depend on a time of day, on products currently being processed and monitored, etc. If the inactivity timer expires (step 809: yes), the training will be triggered in step 810, and the process continues in step 801 by receiving online measurements. Hence the fallow periods are automatically used for training. If the inactivity timer does not expire (step 809: no), the process continues in step 801 by receiving online measurements.

As is evident from the above examples, the training, or any interactive engagement session, may be used as virtual alertness tests, and the training (interactive engagement session) can be used to assess the human operator's reaction times in the virtual alertness tests.

In the above examples it is assumed that an engagement session will not be triggered when anomalies may happen within the predicted period. However, in real life something unpredictable may happen. Further, there may be implementations allowing engagement sessions without checking the stability. For those situations, the artificial intelligent operator, for example the coordination unit, may be configured to perform an example functionality described in Figure 9.

Referring to Figure 9, when it is detected in step 901 that a user input is needed for a control action, it is checked in step 902, whether the human operator is currently involved with an engagement session, for example is training. If the human operator is involved with an engagement session (step 902: yes), the engagement session is immediately stopped in step 903, and the operator will be directed in step 904 via the engagement interface from the engagement interface to a relevant control panel in the human-machine interface, wherein the human operator is prompted in step 905 for user input.

If the human operator is not involved with an engagement session (step 902: no), the human operator is prompted in step 905 for user input.

It should be appreciated that prompting for user input may include displaying one or more suggestions for the input.

The one or more models trained for the AI operator may need retraining or may be retrained just to make sure that they reflect the human operators in the control room as well as possible. Retraining may be triggered based on various triggering events. The retraining may be triggered automatically, by the coordinator, for example, and/or manually, i.e. a human operator inputting a command causing the retraining to be triggered. Examples that may cause the retraining being triggered include a predetermined time limit expiring from the last time the model was trained/retrained, or in response to the amount of stored measurement results of operators increasing by a certain amount or based on some prediction metrics (e.g. MSE (mean squared error) of predictions vs measured values).

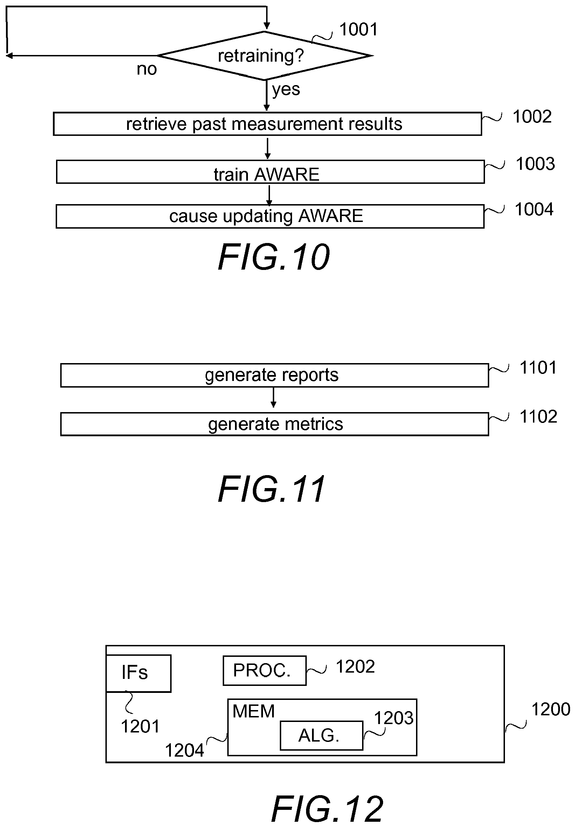

Figure 10 describes an example of a functionality of the trainer unit relating to retraining of the awareness unit (AWARE). With the re-training the unit will be updated to be in more accordance with current human operators and current configurations that may be different than those used in previous training.

Referring to Figure 10, it is monitored in step 1001, whether or not the retraining is triggered. When it is detected that the retraining has been triggered (step 1001: yes), past measurements results are retrieved (re-retrieved) in step 1002 from the local data storage to be used as the training data. When the past measurements results are (re-)retrieved, the measurement results stored to the data storage after the previous training/retraining will be inputted to the training procedure. Then the model (AWARE), based on the deep neural network, for example, is trained in step 1003 to obtain an updated model for the awareness unit. When the retraining process ends, updating of the model to the AI operator is caused in step 1004.

If the retraining does not cause an actual update (i.e. only minor changes, if any) to a corresponding model, there is no need to cause the updating (step 1004).

Naturally, the other models in the other units may undergo a similar retraining process. Further, finalizing training of a pre-trained model also uses the same principles.

In other implementations, in which the trainer unit is part of the online system, the retraining process may be performed continuously (no monitoring of step 1001 being implemented, and the received measurement results may be added to the training material, i.e. also step 1002 may be omitted).

In the above examples it is assumed that there are enough past measurement results that can be used as training data and validation data to create the trained models. Although not explicitly stated, the training process continues until a predetermined accuracy criterion for the predictions is reached. The accuracy criterion depends on the process in an industrial plant for which the model is trained, and the purpose of the model.

Figure 11 illustrates an implementation in which the alertness levels determined are also stored. For example, the AI operator may generate (step 1101) different reports (step 1101) based on the stored alertness levels. Further, the AI operator may generate (step 1102) different performance metrics, or anonymized performance metrics based on the stored alertness levels. A supervisor who is responsible for safety and well-being of the human operators, and scheduling of work may use the reports and performance metrics to schedule working shifts, rotations within a working shift, etc. according to human operators' optimal time-shifts, etc., all being measures that in the long run increase the productivity and shortens shut down times of the process.

The steps and related functions described above in Figures 2 to 11 are in no absolute chronological order, and some of the steps may be performed simultaneously or in an order differing from the given one. Other functions can also be executed between the steps or within the steps. For example, the human operator may have access to ENU and is able to manipulate set point, etc, in the simulated environment. Some of the steps or part of the steps can also be left out or replaced by a corresponding step or part of the step. For example, monitoring inactivity timer expiry may be left out in the process of Figure 8. Further, the described processes may run in parallel, and features from one process (functionality) may be combined to another process (functionality).

The techniques and methods described herein may be implemented by various means so that equipment/a device/an apparatus configured to implement artificial intelligence operator, or create/update one or more trained models according to at least partly on what is disclosed above with any of Figures 1 to 10, including implementing one or more functions/operations of a corresponding equipment/unit described above with an embodiment/example, for example by means of any of Figures 1 to 10, comprises not only prior art means, but also means for implementing the one or more functions/operations of a corresponding functionality described with an embodiment/example, for example by means of any of Figures 1 to 10, and the equipment may comprise separate means for each separate function/operation, or means may be configured to perform two or more functions/operations. For example, one or more of the means and/or the coordinator unit and/or the awareness unit and/or the engagement unit and/or the artificial intelligence unit and/or one or more trainer units and/or one or more pre-trainer units described above may be implemented in hardware (one or more devices), firmware (one or more devices), software (one or more modules), or combinations thereof.

Figure 12 is a simplified block diagram illustrating some units for equipment 1200 configured to provide the artificial intelligence operator equipment, or a corresponding computing device, with the one or more units or corresponding trained models, and/or the offline equipment comprising at least one or more pre-trained units, trainer units, or corresponding units and sub-units, described above with Figures 1 to 11, or corresponding functionality or some of the corresponding functionality if functionalities are distributed in the future. In the illustrated example, the equipment comprises one or more interfaces (IF) 1201 for receiving and/or transmitting information from or to other devices, and from or to a user, including different human-machine interfaces, one or more processors 1202 configured to implement the functionality of the artificial intelligence equipment, or a corresponding computing device, with the coordinator unit and/or the awareness unit and/or the engagement unit and/or the artificial intelligence unit, and/or the offline equipment comprising at least one or more trainer units and/or one or more pre-trainer units, described above with Figures 1 to 11, or at least part of corresponding functionality as a sub-unit functionality if a distributed scenario is implemented, with corresponding algorithms 1203, and one or more memories 1204 usable for storing a computer program code required for the functionality of the artificial intelligence equipment, or a corresponding computing device, with the coordinator unit and/or the awareness unit and/or the engagement unit and/or the artificial intelligence unit, and/or the offline equipment comprising at least one or more trainer units and/or one or more pre-trainer units, i.e. the algorithms for implementing the functionality. The memory 1204 is also usable for storing the one or more trained models, the set of engagement events, and other information, such as alertness levels.

In other words, equipment (device, apparatus) configured to provide the artificial intelligence equipment, or a corresponding computing device, with the coordinator unit and/or the awareness unit and/or the engagement unit and/or the artificial intelligence unit, and/or the offline equipment comprising at least one or more trainer units and/or one or more pre-trainer units, or a device/apparatus configured to provide one or more of the corresponding functionalities described above with Figures 1 to 11, is a computing equipment that may be any apparatus or device or equipment or node configured to perform one or more of the corresponding functionalities described above with an embodiment/example/implementation, and it may be configured to perform functionalities from different embodiments/examples/implementations. Required units and sub-units, for example the awareness unit, may be separate units, even located in another physical apparatus, the distributed physical apparatuses forming one logical equipment providing the functionality, or integrated to another unit in the same equipment.

The equipment configured to provide the artificial intelligence equipment, or a corresponding computing device, with the coordinator unit and/or the awareness unit and/or the engagement unit and/or the artificial intelligence unit, and/or the offline equipment comprising at least one or more trainer units and/or one or more pre-trainer units, or a device configured to provide one or more corresponding functionalities may generally include one or more processors, controllers, control units, micro-controllers, or the like connected to one or more memories and to various interfaces of the equipment. Generally a processor is a central processing unit, but the processor may be an additional operation processor. Each or some or one of the units/sub-units and/or algorithms described herein may be configured as a computer or a processor, or a microprocessor, such as a single-chip computer element, or as a chipset, including at least a memory for providing storage area used for arithmetic operation and an operation processor for executing the arithmetic operation. Each or some or one of the units/sub-units and/or algorithms described above may comprise one or more computer processors, application-specific integrated circuits (ASIC), digital signal processors (DSP), digital signal processing devices (DSPD), programmable logic devices (PLD), field-programmable gate arrays (FPGA), graphics processing units (GPUs), logic gates and/or other hardware components that have been programmed and/or will be programmed by downloading computer program code (one or more algorithms) in such a way to carry out one or more functions of one or more embodiments/implementations/examples. An embodiment provides a computer program embodied on any client-readable distribution/data storage medium or memory unit(s) or article(s) of manufacture, comprising program instructions executable by one or more processors/computers, which instructions, when loaded into a device, constitute the coordinator unit and/or the awareness unit and/or the engagement unit and/or the artificial intelligence unit, and/or the one or more trainer units and/or the one or more pre-trainer units, or any sub-unit. Programs, also called program products, including software routines, program snippets constituting "program libraries", applets and macros, can be stored in any medium and may be downloaded into an apparatus. In other words, each or some or one of the units/sub-units and/or the algorithms described above may be an element that comprises one or more arithmetic logic units, a number of special registers and control circuits.

Further, the artificial intelligence equipment, or a corresponding computing device, with the coordinator unit and/or the awareness unit and/or the engagement unit and/or the artificial intelligence unit, and/or the offline equipment comprising at least one or more trainer units and/or one or more pre-trainer units, or a device configured to provide one or more of the corresponding functionalities described above with Figures 1 to 11 may generally include volatile and/or nonvolatile memory, for example EEPROM, ROM, PROM, RAM, DRAM, SRAM, double floating-gate field effect transistor, firmware, programmable logic, etc. and typically store content, data, or the like. The memory or memories may be of any type (different from each other), have any possible storage structure and, if required, being managed by any database management system. In other words, the memory, or part of it, may be any computer-usable non-transitory medium within the processor/equipment or external to the processor/equipment, in which case it can be communicatively coupled to the processor/equipment via various means as is known in the art. Examples of an external memory include a removable memory detachably connected to the apparatus, a distributed database and a cloud server. The memory may also store computer program code such as software applications (for example, for one or more of the units/sub-units/algorithms) or operating systems, information, data, content, or the like for the processor to perform steps associated with operation of the equipment in accordance with examples/embodiments.

It will be obvious to a person skilled in the art that, as technology advances, the inventive concept can be implemented in various ways. The invention and its embodiments are not limited to the examples described above, but may vary within the scope of the claims.

## Claims

1. A computer implemented method comprising:
receiving (701) online measurement results of a human operator, who is monitoring and controlling an industrial process, the online measurements results comprising at least a delay associated with the human operator's interactions with the industrial process, the interactions including control actions;
receiving (701) online measurement results from the process, the online measurement results from the process indicating process responses to the control actions;
**characterized by**
receiving (701) online measurement results of a control room where the human operator is located;
inputting the measurement results of the human operator, the measurement results of the control room, and at least the measurement results indicating process responses to a first trained model, which utilizes machine learning;
processing (702), by the first trained model, the inputted measurement results to one or more alertness levels;
triggering (705), in response to the alertness level indicating too low level of alertness, an engagement session with the human operator.

2. A computer implemented method as claimed in claim 1, wherein the online measurement results of the human operator further comprise at least one of visual data, audio data and one or more physiological values of the human operator.

3. A computer implemented method as claimed in claim 1 or 2, further comprising:
processing (802) the measurement results from the industrial process to one or more predictions of the state of the process;
determining (603), before triggering the engagement session, stability of the industrial process within a time limit, based on the one or more predictions; and
triggering (705) the engagement session only if the industrial process is predicted to be stable within the time limit.

4. A computer implemented method as claimed in any of the preceding claims, further comprising sending, in response to the alertness level indicating too low level of alertness, a notification of the alertness level to the operator's supervisor and/or to reserve personnel.

5. A computer implemented method as claimed in any of the preceding claims, wherein triggering the engagement session includes prompting a user for acceptance of the session; and starting an engagement session in response to receiving a user input accepting the engagement session.

6. A computer implemented method as claimed in any of the preceding claims, further comprising:
increasing (607), in response to starting an engagement session, automatic anomaly detection from a normal level to a more sensitive level; and
decreasing (610), in response to the engagement session ending, the automatic anomaly detection back to the normal level.

7. A computer implemented method as claimed in any of the preceding claims, wherein the engagement session is a computer-based automated interactive training session relating to the process, a computer instructed physical activity session, a computer narrated audio reporting session about the performance of the operation of the process, a computer narrated visual reporting session about the performance of the operation of the process, an application-based interactive training session with gamification, the training session relating to the process, or any combination thereof.

8. A computer implemented method as claimed in any of the preceding claims, further comprising
storing the alertness levels;
generating (1101) reports based on the stored alertness levels; and
generating (1102) performance metrics or anonymized performance metrics based on the stored alertness levels.

9. A computer implemented method as claimed in any of the preceding claims, further comprising:
receiving (606) a user input requesting triggering an engagement session; and
starting (608) an engagement session in response to the received user input requesting triggering an engagement session.

10. A computer implemented method as claimed in any of the claims 3 to 9, further comprising:
outputting (612), in response to automatically detecting an anomaly in the industrial process, one or more suggestions for one or more control actions for the human operator.

11. A computer program product (1203) comprising program instructions, which, when run on a computing equipment, causes the computing equipment to perform a method as claimed in any preceding claim upon receiving measurement results of a human operator controlling a process in an industrial plant and measurement results measured from the process in the industrial plant and measurement results of a control room where the human operator is located.

12. Computing equipment (133, 1200) comprising means configured for implementing a method as claimed in any of claims 1 to 10.

13. A system (100) comprising at least:
one or more sensors (111) providing measurement results on one or more processes (110) in an industrial plant, the online measurement results from a process (110) indicating process responses to control actions;
a control room (130) for monitoring and controlling the one or more industrial processes (110) by one or more human operators, the control room (130) comprising means for measuring online at least a human operator's interactions with an industrial process (110), the interactions including control actions; and
a computing equipment (133, 1200) as claimed in claim 12.

14. A system (100) as claimed in claim 13, wherein the control room (130) is one of a power plant control room, a process control room, a grid control center, a mine central control room, an oil and/or gas command center, a rail operating center, a traffic management center, a marine fleet handling center, a data center control room, a hospital capacity command center, a control room for a manufacturing plant, a control room for a chemical processing plant, and a control room for a mineral processing plant.

## Patentansprüche

1. Computerimplementiertes Verfahren, umfassend:
Empfangen (701) von Online-Messergebnissen eines menschlichen Bedieners, der einen industriellen Prozess überwacht und steuert, die Online-Messergebnisse umfassend mindestens eine Verzögerung, die mit Interaktionen des menschlichen Bedieners mit dem industriellen Prozess assoziiert ist, wobei die Interaktionen Steuerungsaktionen enthalten;
Empfangen (701) von Online-Messergebnissen von dem Prozess, wobei die Online-Messergebnisse von dem Prozess Prozessreaktionen auf die Steuerungsaktionen angeben;
**gekennzeichnet durch**
Empfangen (701) von Online-Messergebnissen einer Steuerwarte, in dem sich der menschliche Bediener befindet;
Eingeben der Messergebnisse des menschlichen Bedieners, der Messergebnisse der Steuerwarte und mindestens der Messergebnisse, die Prozessreaktionen auf ein erstes trainiertes Modell, das Maschinenlernen nutzt, angeben;
Verarbeiten (702), durch das erste trainierte Modell, der eingegebenen Messergebnisse zu einer oder mehreren Aufmerksamkeitsstufen;
Auslösen (705), als Reaktion auf die Aufmerksamkeitsstufe, die eine zu niedrige Stufe der Aufmerksamkeit angibt, einer Eingriffssitzung mit dem menschlichen Bediener.

2. Computerimplementiertes Verfahren nach Anspruch 1, wobei die Online-Messergebnisse des menschlichen Bedieners ferner mindestens eines von visuellen Daten, Audiodaten und eines oder mehrerer physiologischer Werte des menschlichen Bedieners umfassen.

3. Computerimplementiertes Verfahren nach Anspruch 1 oder 2, ferner umfassend:
Verarbeiten (802) der Messergebnisse von dem industriellen Prozess zu einer oder mehreren Vorhersagen des Zustands des Prozesses;
Bestimmen (603), vor dem Auslösen der Eingriffssitzung, der Stabilität des industriellen Prozesses innerhalb einer Zeitbegrenzung basierend auf der einen oder den mehreren Vorhersagen; und
Auslösen (705) der Eingriffssitzung nur dann, wenn vorhergesagt wird, dass der industrielle Prozess innerhalb der Zeitbegrenzung stabil sein wird.

4. Computerimplementiertes Verfahren nach einem der vorhergehenden Ansprüche, ferner umfassend Senden, als Reaktion auf die Aufmerksamkeitsstufe, die eine zu niedrige Stufe der Aufmerksamkeit angibt, einer Benachrichtigung über die Aufmerksamkeitsstufe an den Vorgesetzten des Bedieners und/oder an Reservepersonal.

5. Computerimplementiertes Verfahren nach einem der vorhergehenden Ansprüche, wobei Auslösen der Eingriffssitzung enthält, einen Benutzer zum Annehmen der Sitzung aufzufordern; und Starten einer Eingriffssitzung als Reaktion auf Empfangen einer Benutzereingabe, die die Eingriffssitzung annimmt.

6. Computerimplementiertes Verfahren nach einem der vorhergehenden Ansprüche, ferner umfassend:
Erhöhen (607), als Reaktion auf Starten einer Eingriffssitzung, einer automatischen Anomaliedetektion von einer normalen Stufe zu einer empfindlicheren Stufe; und
Verringern (610), als Reaktion darauf, dass die Eingriffssitzung endet, der automatischen Anomaliedetektion zurück auf die normale Stufe.

7. Computerimplementiertes Verfahren nach einem der vorhergehenden Ansprüche, wobei die Eingriffssitzung eine computerbasierte automatisierte interaktive Trainingssitzung im Zusammenhang mit dem Prozess, eine computerangeleitete Sitzung körperlicher Aktivität, eine computervorgetragene Audioberichterstattungssitzung über die Durchführung des Betriebs des Prozesses, eine computervorgetragene visuelle Berichterstattungssitzung über die Durchführung des Betriebs des Prozesses, eine anwendungsbasierte interaktive Trainingssitzung mit Gamifizierung, wobei die Trainingssitzung mit dem Prozess im Zusammenhang steht, oder eine beliebige Kombination davon ist.

8. Computerimplementiertes Verfahren nach einem der vorhergehenden Ansprüche, ferner umfassend
Speichern der Aufmerksamkeitsstufen;
Erzeugen (1101) von Berichten basierend auf den gespeicherten Aufmerksamkeitsstufen; und
Erzeugen (1102) von Leistungsmetriken oder anonymisierten Leistungsmetriken basierend auf den gespeicherten Aufmerksamkeitsstufen.

9. Computerimplementiertes Verfahren nach einem der vorhergehenden Ansprüche, ferner umfassend:
Empfangen (606) einer Benutzereingabe, die Auslösen einer Eingriffssitzung anfordert; und
Starten (608) einer Eingriffssitzung als Reaktion auf die empfangene Benutzereingabe, die Auslösen einer Eingriffssitzung anfordert.

10. Computerimplementiertes Verfahren nach einem der Ansprüche 3 bis 9, ferner umfassend:
Ausgeben (612), als Reaktion auf automatisches Detektieren einer Anomalie in dem industriellen Prozess, eines oder mehrerer Vorschläge für eine oder mehrere Steuerungsaktionen für den menschlichen Bediener.

11. Computerprogrammprodukt (1203) umfassend Protokollanweisungen, die, wenn sie auf einer Berechnungsausrüstung ausgeführt werden, die Berechnungsausrüstung veranlassen, ein Verfahren nach einem der vorhergehenden Ansprüche nach Empfangen von Messergebnissen eines menschlichen Bedieners, der einen Prozess in einem industriellen Werk steuert, und von Messergebnissen, die von dem Prozess in dem industriellen Werk gemessen werden, und von Messergebnissen einer Steuerwarte, in dem sich der menschliche Bediener befindet, durchzuführen.

12. Berechnungsausrüstung (133, 1200), umfassend Mittel zum Implementieren eines Verfahrens nach einem der Ansprüche 1 bis 10.

13. System (100), umfassend mindestens:
einen oder mehrere Sensoren (111), die Messergebnisse über einen oder mehrere Prozesse (110) in einem industriellen Werk bereitstellen, wobei die Online-Messergebnisse von einem Prozess (110) Prozessreaktionen auf Steuerungsaktionen angeben;
eine Steuerwarte (130) zum Überwachen und Steuern des einen oder der mehreren industriellen Prozesse (110) durch einen oder mehrere menschliche Bediener, die Steuerwarte (130) umfassend Mittel zum Online-Messen der Interaktionen mit einem industriellen Prozess (110) mindestens eines menschlichen Bedieners, wobei die Interaktionen Steuerungsaktionen enthalten; und
eine Berechnungsausrüstung (133, 1200) nach Anspruch 12.

14. System (100) nach Anspruch 13, wobei die Steuerwarte (130) eine einer Kraftwerk-Steuerwarte, einer Prozesssteuerwarte, einer Netzleitstelle, einer zentralen Grubensteuerwarte, einer Öl- und/oder Gasleitstelle, einer Bahnbetriebszentrale, einer Verkehrsleitzentrale, einer Schiffsflotten-Abfertigungszentrale, einer Datenzentrum-Steuerwarte, eine Krankenhauskapazität-Leitstelle, einer Steuerwarte für eine Produktionsanlage, einer Steuerwarte für eine chemische Verarbeitungsanlage und einer Steuerwarte für eine Mineralienverarbeitungsanlage ist.

## Revendications

1. Procédé mis en œuvre par ordinateur comprenant :
la réception (701) de résultats de mesure en ligne d'un opérateur humain, qui surveille et contrôle un processus industriel, les résultats de mesure en ligne comprenant au moins un retard associé aux interactions de l'opérateur humain avec le processus industriel, les interactions comprenant des actions de contrôle ;
la réception (701) de résultats de mesure en ligne en provenance du processus, les résultats de mesure en ligne provenant du processus indiquant des réponses du processus aux actions de contrôle ;
**caractérisé par**
la réception (701) de résultats de mesure en ligne d'une salle de contrôle où se trouve l'opérateur humain ;
la saisie des résultats de mesure de l'opérateur humain, des résultats de mesure de la salle de contrôle, et au moins des résultats de mesure indiquant des réponses du processus dans un premier modèle d'apprentissage, qui utilise un apprentissage automatique ;
le traitement (702), par le premier modèle d'apprentissage, des résultats de mesure saisis en un ou plusieurs niveaux de vigilance ;
le déclenchement (705), en réponse au fait que le niveau de vigilance indique un niveau de vigilance trop faible, d'une session d'engagement avec l'opérateur humain.

2. Procédé mis en œuvre par ordinateur selon la revendication 1, dans lequel les résultats de mesure en ligne de l'opérateur humain comprennent en outre au moins l'une de données visuelles, de données audio et d'une ou plusieurs valeurs physiologiques de l'opérateur humain.

3. Procédé mis en œuvre par ordinateur selon la revendication 1 ou 2, comprenant en outre :
le traitement (802) des résultats de mesure provenant du processus industriel en une ou plusieurs prédictions de l'état du processus ;
la détermination (603), avant de déclencher la session d'engagement, de la stabilité du processus industriel en-deçà d'une limite de temps, sur la base desdites une ou plusieurs prédictions ; et
le déclenchement (705) de la session d'engagement uniquement s'il est prédit que le processus industriel sera stable en-deçà de la limite de temps.

4. Procédé mis en œuvre par ordinateur selon l'une quelconque des revendications précédentes, comprenant en outre l'envoi, en réponse au fait que le niveau de vigilance indique un niveau de vigilance trop faible, d'une notification du niveau de vigilance au superviseur de l'opérateur et/ou au personnel de réserve.

5. Procédé mis en œuvre par ordinateur selon l'une quelconque des revendications précédentes, dans lequel le déclenchement de la session d'engagement comprend l'invitation d'un utilisateur à accepter la session ; et le démarrage d'une session d'engagement en réponse à la réception d'une saisie de l'utilisateur acceptant la session d'engagement.

6. Procédé mis en œuvre par ordinateur selon l'une quelconque des revendications précédentes, comprenant en outre :
l'augmentation (607), en réponse au démarrage d'une session d'engagement, d'une détection automatique d'anomalie, d'un niveau normal à un niveau plus sensible ; et
la diminution (610), en réponse à la fin de la session d'engagement, de la détection automatique d'anomalie pour la ramener au niveau normal.

7. Procédé mis en œuvre par ordinateur selon l'une quelconque des revendications précédentes, dans lequel la session d'engagement est une session d'apprentissage interactive automatisée sur ordinateur concernant le processus, une séance d'activité physique assistée par ordinateur, une session de compte-rendu audio dit sur ordinateur concernant la réalisation de l'opération du processus, une session de compte-rendu visuel dit sur ordinateur concernant la réalisation de l'opération du processus, une session d'apprentissage interactive basée sur une application avec ludification, la session d'apprentissage concernant le processus, ou toute combinaison de ceux-ci.

8. Procédé mis en œuvre par ordinateur selon l'une quelconque des revendications précédentes, comprenant en outre
le stockage des niveaux de vigilance ;
la génération (1101) de comptes-rendus basés sur les niveaux de vigilance stockés ; et
la génération (1102) de métriques de performances ou de métriques de performances anonymisées sur la base des niveaux de vigilance stockés.

9. Procédé mis en œuvre par ordinateur selon l'une quelconque des revendications précédentes, comprenant en outre :
la réception (606) d'une saisie de l'utilisateur demandant le déclenchement d'une session d'engagement ; et
le démarrage (608) d'une session d'engagement en réponse à la saisie de l'utilisateur reçue demandant le déclenchement d'une session d'engagement.

10. Procédé mis en œuvre par ordinateur selon l'une quelconque des revendications 3 à 9, comprenant en outre :
la fourniture en sortie (612), en réponse à la détection automatique d'une anomalie dans le processus industriel, d'une ou plusieurs suggestions concernant une ou plusieurs actions de contrôle pour l'opérateur humain.

11. Produit de programme informatique comprenant des instructions de programme qui, lorsqu'elles sont exécutées sur un équipement informatique, amènent l'équipement informatique à mettre en œuvre un procédé selon l'une quelconque des revendications précédentes lors de la réception de résultats de mesure d'un opérateur humain contrôlant un processus dans une installation industrielle et de résultats de mesure provenant du processus dans l'installation industrielle et de résultats de mesure d'une salle de contrôle où se trouve l'opérateur humain.

12. Équipement informatique (133, 1200) comprenant un moyen destiné à mettre en œuvre un procédé selon l'une quelconque des revendications 1 à 10.

13. Système (100) comprenant au moins :
un ou plusieurs capteurs (111) fournissant des résultats de mesure concernant un ou plusieurs processus (110) dans une installation industrielle, les résultats de mesure en ligne provenant d'un processus (110) indiquant des réponses du processus à des actions de contrôle ;
une salle de contrôle (130) destinée à surveiller et contrôler lesdits un ou plusieurs processus industriels (110) par un ou plusieurs opérateurs humains, la salle de contrôle (130) comprenant un moyen destiné à mesurer en ligne des interactions d'au moins un opérateur humain avec un processus industriel (110), les interactions comprenant des actions de contrôle ; et
un équipement informatique (133, 1200) selon la revendication 12.

14. Système (100) selon la revendication 13, dans lequel la salle de contrôle (130) est l'un d'une salle de contrôle d'une centrale électrique, d'une salle de contrôle de processus, d'un centre de contrôle du réseau, d'une salle de contrôle centrale de mine, d'un centre de commandement pour le pétrole et/ou le gaz, d'un centre d'exploitation ferroviaire, d'un centre de gestion du trafic, d'un centre de gestion de flotte maritime, d'une salle de contrôle de centre de données, d'un centre de commandement de capacité d'un hôpital, d'une salle de contrôle destinée à une usine de fabrication, d'une salle de contrôle destinée à une usine de traitement chimique, et d'une salle de contrôle destinée à une usine de traitement de minéraux.
